# FASCICULE DE BREVET EUROPEEN

(11) **EP 2 986 539 B1**
(45) Date de publication et mention de la délivrance du brevet: **06.06.2018**
(21) Numéro de dépôt: 14722718.5
(22) Date de dépôt: 15.04.2014
(51) Int. Cl.: B05B 11/00, A61M 15/08, B65D 83/38, B65D 83/22

(54) **FLACON DE CONDITIONNEMENT DE LIQUIDE**
FLASCHE ZUM VERPACKEN VON FLÜSSIGKEITEN
BOTTLE FOR PACKAGING LIQUID

(30) Priorité: 16.04.2013 FR 1300893
(43) Date de publication de la demande: 24.02.2016
(73) Titulaire: Laboratoires THEA, 63100 Clermont-Ferrand (FR)
(72) Inventeur: DEFEMME, Alain, 63400 Chamalières (FR); MERCIER, Fabrice, 63000 Clermont-ferrand (FR)
(74) Mandataire: Santarelli
(86) Numéro de dépôt international: PCT/IB2014/000546
(87) Numéro de publication internationale: WO 2014/170736

(56) Documents cités:
- WO-A1-03/066235
- WO-A1-2009/065599
- WO-A1-2010/139883
- FR-A1- 2 927 552
- US-A1- 2010 095 957

## Description

La présente invention concerne un flacon de distribution de liquide, et plus particulièrement un flacon de distribution équipé d'un système de pompe pour amorcer la distribution de ce liquide.

L'invention trouve particulièrement utilisation pour des flacons de distribution de liquides ophtalmiques en goutte à goutte, mais il sera compris qu'elle s'applique pour la distribution de tout type de liquide.

Dans ce type de flacon, on utilise de façon connue un mécanisme à pompe que l'on actionne en pressant une partie mobile du dispositif. Afin de concevoir un flacon à l'ergonomie optimale, qui facilite la prise en main et qui permette d'appliquer sur la partie mobile l'effort nécessaire à l'activation de la pompe, il est connu de l'équiper d'une coiffe pour aider à la manoeuvre de la partie mobile et donc de la pompe. Une telle coiffe comporte un corps cylindrique qui vient recouvrir au moins en partie le réservoir du flacon, et qui présente à son sommet une paroi supérieure plane percée en son centre pour permettre le passage de l'embout diffuseur du flacon. Lorsque la coiffe est en place sur le flacon, la paroi supérieure vient prendre appui contre la partie mobile de la pompe. On augmente ainsi l'étendue de la zone sur laquelle on appuie pour actionner la pompe et on facilite son fonctionnement à long terme.

Différentes variantes de coiffes peuvent être utilisées avec le flacon pour faciliter l'actionnement de la pompe, en prenant par exemple la forme telle que revendiquée dans le brevet WO 2010/139883 déposée par la demanderesse, qui décrit une coiffe en deux parties adaptées à coulisser l'une vers l'autre parallèlement à l'axe de la pompe et qui présentent chacune une ailette latérale de préhension, qui facilite encore l'actionnement des parties du poussoir. Ce document décrit un flacon selon le préambule de la revendication 1. Les flacons de distribution de liquide sont communément utilisés pour la distribution de collyre ou d'autres substances pour des soins oculaires. Il est courant, notamment du fait du nombre de flacons vendus ensemble dans une même boîte, que plusieurs flacons soient simultanément à disposition de l'utilisateur alors qu'il est important qu'ils ne soient pas mis en usage au même moment, mais plutôt l'un après l'autre. A titre d'exemple, on peut préconiser que les flacons, qui sont produits sous conditionnement stérile, doivent être conservés à une première température avant ouverture et qu'ils peuvent après ouverture être conservés plusieurs semaines à température ambiante. A l'origine de l'invention, il est donc apparu souhaitable d'améliorer les dispositifs de distribution tels qu'ils ont été décrits précédemment afin de permettre une identification claire de la première utilisation ou non du dispositif, puisque après ce premier usage, le dispositif ne peut être conservé que dans une durée définie règlementairement.

Dans ce contexte, la présente invention propose un flacon de distribution de liquide comportant une coiffe qui permette un marquage simple et efficace visant à prévenir l'utilisateur de la première utilisation du flacon.

A cet effet, l'invention propose un flacon de conditionnement selon la revendication 1. Ainsi, l'utilisateur peut facilement constater si le flacon qu'il a en main est déjà ouvert ou non, par une constatation visuelle notamment de la fracturation du dispositif de retenue de la partie amovible. L'utilisateur évite ainsi d'ouvrir tous les flacons en même temps et il peut conserver chacun des flacons selon les préconisations d'usage.

Lorsque le manchon recouvre la collerette de la portion mobile, la paroi transversale est adaptée à reposer contre la face supérieure de la collerette et l'alésage central permet le passage de l'embout de distribution. Par ailleurs, on assure par l'intermédiaire de la coiffe, outre sa fonction de témoin de première utilisation, une fonction d'actionnement de la pompe par une partie venant recouvrir la collerette de la portion mobile de la pompe.

Selon une caractéristique de l'invention, des moyens d'encliquetage sont disposés sur la face intérieure de la paroi latérale pour réaliser l'emprisonnement de la collerette entre ces moyens d'encliquetage et la paroi transversale. Ainsi, on s'assure que la coiffe ne se désengage pas du flacon, sauf action intentionnelle de l'utilisateur. La coiffe est laissée à demeure sur le flacon et seule la partie amovible peut être retirée. On s'assure ainsi que la fonction de témoin de première utilisation dépendra de la présence ou non de la partie amovible, ou de la rupture ou non du dispositif de retenue.

Dans des modes de réalisation préférés de l'invention, la paroi transversale du manchon est prolongée radialement par une ailette de préhension, qui s'étend vers l'extérieur du manchon, et la paroi latérale cylindrique de ce manchon s'étend axialement pour recouvrir la collerette et au moins une partie du réservoir. Avantageusement, cette paroi latérale du manchon de recouvrement s'étend axialement le long du réservoir sous cette ailette. L'ailette de préhension sert à faciliter la poussée axiale sur le manchon pour l'actionnement de la portion mobile de la pompe, et la paroi latérale sert à faciliter le coulissement du manchon le long du flacon, sa position préférée sous l'ailette permettant de diminuer l'effet de basculement et les frottements que peuvent générer le coulissement du manchon le long du flacon.

Selon une caractéristique de l'invention, on prévoit des moyens de blocage en rotation de la coiffe relativement au flacon, afin de figer la position relative angulaire de la coiffe. Dans cette position angulaire bloquée de la coiffe, la paroi latérale du manchon de recouvrement s'étend le long du réservoir, sous l'ailette de préhension, et sur un côté du flacon opposé au côté réservé à l'étiquetage.

Ainsi, la partie amovible est adaptée à recouvrir l'embout de distribution. Cette partie amovible est rendue solidaire de la paroi transversale par des picots de maintien fracturables par rotation de la partie amovible par rapport au manchon lors de la première utilisation. Avantageusement, ces picots sont disposés transversalement entre l'extrémité de la partie amovible et le bord délimitant l'alésage central, dans le plan de la collerette. Ainsi, leur disposition dans le prolongement de la collerette facilite le démoulage de l'ensemble.

La partie amovible de la coiffe peut alors présenter une forme conique complémentaire de celle de l'embout de distribution pour venir le recouvrir, et elle peut comporter des bossages sur la face intérieure de la paroi délimitant ladite forme conique. Ainsi, on diminue la section interne de la partie amovible, ce qui permet, lorsque la coiffe est mise en place, l'accrochage par la partie amovible de l'embout ou d'un capuchon rapporté initialement sur l'embout. On facilite ainsi la manipulation et l'enlèvement du capuchon par le pinçage de ce dernier par la partie amovible.

Selon des caractéristiques de l'invention, l'embout conique formant la partie amovible de la coiffe peut présenter une forme de portion de cône, tronqué à l'extrémité opposée de la paroi transversale du manchon de recouvrement, pour laisser passage au capuchon. La portion de cône présente sur sa face externe des nervures axiales de rigidification facilitant sa préhension et l'action exercée par l'utilisateur.

Ce type de partie amovible disposée au-dessus de la paroi transversale est intéressant dans le cadre d'un mode de réalisation particulier de l'invention où le manchon de recouvrement comporte deux parties coulissantes l'une par rapport à l'autre. Une partie supérieure recouvre la portion mobile pour l'actionnement de la pompe et une partie inférieure recouvre le fond du réservoir, et les deux parties sont bloquées en rotation relative l'une par rapport à l'autre. Le rapprochement de ces parties l'une vers l'autre génère l'actionnement de la pompe, et les deux parties présentent chacune une ailette radiale de préhension du flacon pour faciliter la manipulation du flacon pour réaliser ce rapprochement. Lorsque l'on souhaite fracturer le dispositif de retenue lors de la première utilisation, le manchon de recouvrement est bloqué en rotation relative par rapport au couple que l'on exerce sur la partie amovible, quel que soit l'endroit de la préhension de l'utilisateur. Si celui-ci tient la partie supérieure, l'utilisateur procède facilement à la fracture du dispositif de retenue. Si au contraire l'utilisateur tient la partie inférieure, la partie supérieure est bloquée en rotation par l'action combinée de la préhension de l'utilisateur et des moyens de blocage en rotation des deux parties entre elles.

L'invention concerne également un procédé de fabrication d'un flacon de conditionnement de liquide selon la revendication 13. Ainsi, on peut fabriquer et procéder au montage de l'ensemble dans différents lieux sans contrevenir aux exigences de stérilité de la fabrication. La coiffe pourra être montée en fin de process dans des conditions non stériles, alors que le flacon aura au préalable été rempli puis fermé hermétiquement par le capuchon dans des conditions stériles.

A cet effet, la coiffe présente une dimension interne adaptée pour que la coiffe puisse recouvrir l'embout de distribution lorsqu'il est lui-même recouvert par un capuchon. On facilite ainsi la mise en oeuvre du dispositif en respectant les conditions de stérilité nécessaire à l'utilisation d'un dispositif de délivrance de gouttes ophtalmiques, sans toutefois imposer les conditions stériles à la fabrication et à la mise en place du poussoir. Le flacon avec son embout de distribution et son capuchon sera réalisé dans des conditions stériles et on viendra équiper par la suite le flacon, avec la coiffe qui vient recouvrir le capuchon.

L'invention et les avantages qui en découlent vont être détaillées dans la description du mode de réalisation qui suit, à l'appui des figures parmi lesquelles :
- la figure 1 est une vue en perspective éclatée d'un flacon de conditionnement de liquide, muni d'une coiffe servant de témoin de première utilisation, selon un premier mode de réalisation de l'invention ;
- la figure 2 est une vue du flacon illustré sur la figure 1 assemblé et en coupe ;
- la figure 3 est une vue en perspective d'un flacon de conditionnement de liquide, muni d'une coiffe servant de témoin de première utilisation, selon un deuxième mode de réalisation de l'invention ;
- la figure 4 est une vue de face d'un flacon selon un troisième mode de réalisation de l'invention ;
- la figure 5 est une vue de côté du flacon illustré sur la figure 4 ;
- la figure 6 est une vue en perspective d'un premier élément formant la coiffe équipant le flacon illustré sur les figures 4 et 5 ; et
- la figure 7 est une vue en perspective d'un deuxième élément formant la coiffe équipant le flacon illustré sur les figures 4 et 5.

Tel qu'illustré sur les figures, le flacon de conditionnement de liquide selon l'invention comporte un réservoir 2, une tête de distribution 4 montée sur le réservoir, et une coiffe 6.

Le réservoir présente une paroi latérale 8 de forme sensiblement cylindrique, ainsi qu'une paroi de fond 10. Le liquide à délivrer en goutte à goutte est stocké dans le réservoir, et il est prélevé par l'intermédiaire de la tête de distribution.

A cet effet, la tête de distribution comporte un dispositif de pompe et un embout de distribution 12 disposé en sortie du dispositif de pompe, de manière à ce que le liquide prélevé dans le réservoir grâce au dispositif de pompe traverse l'embout par un canal central non représenté jusqu'à un orifice terminal d'expulsion de liquide 14. Le dispositif de pompe comporte dans la partie visible représentée sur les figures une portion mobile 16 adaptée à se déplacer axialement par rapport au réservoir pour créer une dépression adaptée pour aspirer le liquide. On déplace la portion mobile en exerçant un effort axial sur une collerette 18 que présente la portion mobile. Cette collerette, qui présente un diamètre au moins égal à celui du réservoir, est prolongée axialement par l'embout de distribution par lequel les gouttes sont délivrées.

La coiffe comporte un manchon de recouvrement 20, une partie amovible 22 et un dispositif de retenue 24 adapté à être fracturé pour délivrer la partie amovible et l'éloigner du manchon de recouvrement.

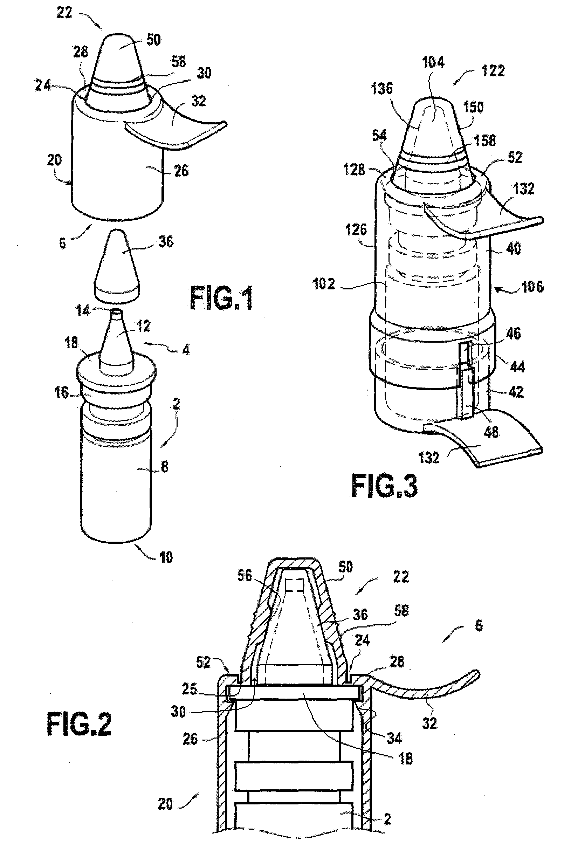

Le dispositif de retenue est formé ici par des picots fracturables 25 (visibles sur la figure 2), dimensionnés pour dans un premier temps rendre solidaires la partie amovible et le manchon de recouvrement et dans un deuxième temps permettre leur séparation par fracture des picots sous un effort de rotation fourni par l'utilisateur.

Le manchon de recouvrement 20 présente une paroi latérale 26 de forme cylindrique, dont une première extrémité axiale est prolongée transversalement vers le centre du manchon pour former une paroi transversale 28, munie d'un alésage central 30. Le manchon de recouvrement est ouvert à la deuxième extrémité opposée.

Lorsque le manchon est en place sur le flacon, la paroi transversale 28 repose en appui sur la face supérieure de la collerette 18. Cette paroi transversale permet d'augmenter la surface d'appui disponible pour l'utilisateur pour agir sur la collerette. Une ailette radiale 32, facilitant la préhension de la coiffe, prolonge radialement cette paroi transversale, vers l'extérieur du manchon, en augmentant encore la surface d'appui disponible. Avantageusement, l'ailette présente une courbure adaptée à faciliter la position du pouce de l'utilisateur.

La paroi latérale du manchon de recouvrement s'étend axialement pour recouvrir la portion mobile et au moins une partie du réservoir lorsque le manchon est en place sur le flacon. On pourra prévoir dans une variante non représentée que la paroi latérale s'étende axialement de façon non uniforme sur le pourtour du flacon et qu'un dégagement axial soit prévu pour permettre la bonne lecture d'une étiquette informative collée sur le réservoir. Le flacon peut comporter alors des moyens de blocage en rotation de la coiffe relativement au flacon pour s'assurer que, dans la position angulaire bloquée de la coiffe, l'étiquette est dégagée et bien visible. Dans une telle variante, on prévoira que la zone de la paroi latérale du manchon de recouvrement qui ne comporte pas le dégagement, c'est à dire la plus étendue, s'étendra le long du réservoir sous l'ailette de préhension.

La coiffe comporte des moyens d'encliquetage 34 disposés sur la face interne de ladite paroi latérale du manchon, pour l'emprisonnement de la collerette entre ces moyens d'encliquetage et la paroi transversale de la coiffe. Tel qu'illustré sur la figure 2, ces moyens d'encliquetage peuvent prendre la forme d'un plan incliné réduisant le diamètre interne du manchon en facilitant, lorsque l'on enfile le manchon autour du flacon, le glissement de la collerette vers une position de blocage entre la paroi transversale et l'épaulement droit des moyens d'encliquetage formé en bout du plan incliné.

La partie amovible 22 présente la forme d'un embout conique 50, disposé sur une face supérieure 52 de la paroi transversale, à l'opposé de la paroi latérale 26 du manchon de recouvrement. Cette partie amovible est disposée sensiblement au centre de la paroi transversale 28, de manière à recouvrir l'alésage central, et elle est maintenue à la paroi supérieure par les picots fracturables 25 qui forment le dispositif de retenue 24. Les picots fracturables sont ici disposés transversalement entre l'extrémité de la partie amovible et le bord délimitant l'alésage central, dans le plan de la collerette. Cette disposition facilite le démoulage de l'ensemble.

La forme conique de la partie amovible est complémentaire de la forme de l'embout de distribution, et de façon avantageuse, complémentaire de la forme et des dimensions d'un capuchon 36 rapporté sur l'embout de distribution préalablement à l'assemblage final du flacon.

Lorsque la coiffe est enfilée autour du flacon par l'extrémité ouverte de la paroi latérale du manchon, la paroi transversale vient au contact de la face supérieure de la collerette, l'alésage central permettant le passage de l'embout de distribution, et la partie amovible de la coiffe vient prendre position autour de l'embout de distribution 12 et du capuchon 36 le recouvrant à l'origine.

La paroi délimitant la forme conique de la partie amovible présente sur sa face interne des bossages 56. Lorsque la coiffe est enfilée autour du flacon, et que la partie amovible recouvre le capuchon disposé sur l'embout de distribution, ces bossages, qui réduisent localement le diamètre interne de la partie amovible, viennent comprimer le capuchon. De la sorte, lorsque l'on manipule la coiffe pour la première fois, et qu'on fracture le dispositif de retenue en tournant la partie amovible par rapport au manchon de recouvrement, on entraîne par friction le capuchon à l'intérieur de la partie amovible, si bien que lorsque la partie amovible est retirée, le capuchon est retiré également et reste à l'intérieur de la coiffe. On pourra prévoir des moyens de préhension sur la face externe de la partie amovible, tels que les stries 58 illustrées à titre d'exemple sur la figure 3, de telle sorte que l'on facilite par l'intermédiaire de cette partie amovible la manipulation du capuchon.

Le montage de la coiffe se réalise en enfilant le flacon à travers l'extrémité libre ouverte du manchon 20, et ce jusqu'à ce que la paroi transversale 28 de ce manchon entre en butée contre la collerette de la pompe, tel que cela est visible sur la figure 3. L'embout de distribution du flacon 12, ainsi qu'un capuchon 36 éventuellement rapporté sur l'embout, passe alors à travers l'alésage central de la paroi transversale. Le flacon est retenu axialement par les moyens d'encliquetage 34.

On a représenté sur les figures 1 et 2 un flacon recouvert par une coiffe particulière en ce que le manchon est monopièce, et en ce que, conformément à chacun des modes de réalisation de l'invention, il présente une face supérieure sur laquelle est rapportée une partie amovible.

On décrit maintenant un deuxième mode de réalisation illustré sur la figure 3. Le flacon de conditionnement présentant dans ce deuxième mode de réalisation une structure proche de celle du flacon décrit ci-dessus, on emploie pour la description à venir des éléments semblables les mêmes références numériques, auxquelles on a ajouté 100.

Le flacon de conditionnement comporte un réservoir 102, une tête de distribution 104 montée sur le réservoir, et une coiffe 106. Dans ce mode de réalisation, la coiffe 106 présente un manchon de recouvrement spécifique.

Le manchon de recouvrement est formé de deux parties 40, 42 adaptées à coulisser axialement le long du réservoir du flacon, l'une vers l'autre lorsqu'on appuie sur les ailettes radiales 132 que chaque partie présente de façon similaire au mode de réalisation précédent. Le rapprochement des deux parties génère l'activation du système de pompe et la délivrance de la goutte. On comprend que les ailettes radiales sont incurvées pour l'ergonomie de la préhension, et que ces courbures sont orientées dans des sens différents puisque pour le rapprochement des deux parties l'une vers l'autre, celles-ci coulissent dans des sens opposés.

On définit ici une partie supérieure 40 du manchon de recouvrement et une partie inférieure 42 par leur position relative lorsque le flacon est posé verticalement tel qu'illustré sur la figure 2.

La partie supérieure du manchon de recouvrement 40 comporte une paroi latérale 43 dont l'extrémité supérieure est prolongée transversalement par une paroi transversale 128 et dont l'extrémité inférieure est prolongée axialement par un collet 44, de manière à présenter un diamètre externe plus important que le diamètre externe du reste de la partie supérieure. Le collet est creusé afin de laisser passage entre la paroi et le flacon pour la paroi latérale de la partie inférieure du manchon de recouvrement 42.

Une rainure 46 est réalisée axialement sur le collet de la partie supérieure et un ergot 48 est réalisé axialement sur la paroi latérale de la partie inférieure, de manière à former ainsi des moyens de guidage axial des deux parties du manchon de recouvrement, en coulissement l'une par rapport à l'autre. On observe qu'ils forment en outre des moyens de blocage en rotation de la partie supérieure par rapport à la partie inférieure.

Tel qu'illustré sur la figure 3, la partie amovible 122 prend la forme d'un embout conique 150, rapporté sur la paroi transversale 128 de la partie supérieure 40 du manchon de recouvrement, de manière à recouvrir comme précédemment l'embout de distribution, et de façon avantageuse, le capuchon 136 rapporté sur l'embout de distribution préalablement à l'assemblage final du flacon.

La partie amovible pourra être différente de celle décrite précédemment mais il sera intéressant que là encore elle comporte des stries 158 sur sa face externe et au moins un bossage sur sa face interne.

La combinaison d'un manchon de recouvrement en deux parties et de la partie amovible entourant l'embout de distribution permet comme dans le mode de réalisation précédent une bonne prise en main lorsque l'on désolidarise la partie amovible.

En effet, on ne pourra désolidariser la partie amovible que si l'on tourne celle-ci et que l'on maintient en position le manchon de recouvrement de la coiffe. Dans le premier mode de réalisation, l'utilisateur comprendra qu'il doit placer une première main sur la partie amovible et sa deuxième main sur le manchon de recouvrement, et non sur le réservoir du flacon dans le cas où celui-ci est accessible. Et dans le deuxième mode de réalisation, quel que soit l'endroit où l'utilisateur place sa deuxième main, le flacon ne pourra tourner dans le vide du fait de l'ergot qui bloque la rotation d'une partie de la coiffe par rapport à l'autre, pour les guider uniquement axialement.

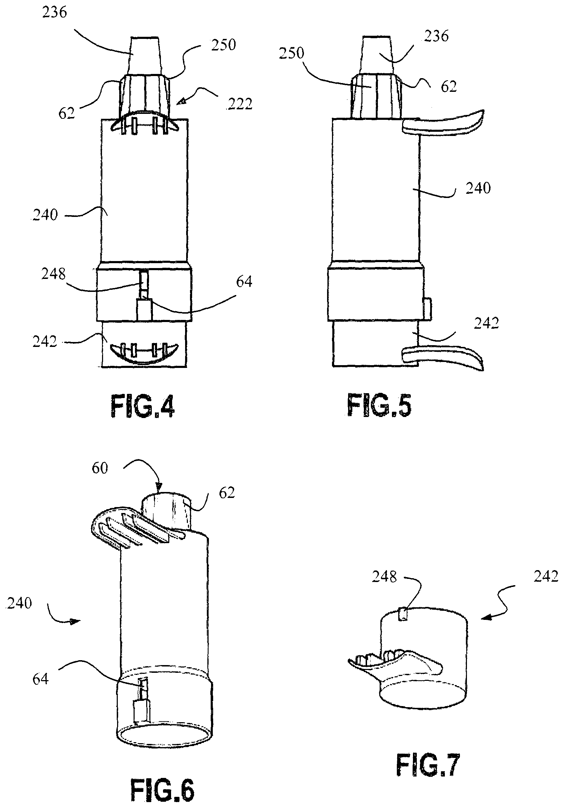

On va maintenant décrire un troisième mode de réalisation illustré sur les figures 4 à 7. Le flacon de conditionnement présentant dans ce troisième mode de réalisation une structure proche de celle des flacons décrit ci-dessus, on emploie pour la description à venir des éléments semblables les mêmes références numériques, auxquelles on a ajouté 200.

Le flacon selon ce mode de réalisation diffère principalement des précédents modes de réalisation par la forme de la partie amovible 222. Celle-ci présente encore une forme d'embout conique 250, spécifique ici en ce qu'il est partiel, c'est à dire qu'il est ouvert à son extrémité 60 opposée au manchon, de manière à ne former qu'une portion de cône. Lorsque la coiffe est rapportée sur le flacon, l'embout 236 dépasse de l'embout conique.

La partie amovible 222 diffère en outre en ce que l'embout comporte des nervures de rigidification 62 qui s'étendent axialement sur le pourtour de l'embout. Ces nervures facilitent la prise en main de la partie amovible et elles facilitent la transmission de l'effort à fournir par l'utilisateur pour la fracture des picots de retenue, non visibles sur les figures 4 à 7. Par ailleurs, la disposition axiale des nervures tend à inciter l'utilisateur à réaliser une rotation de l'embout conique, et le fait que ces nervures s'étendent uniquement sur une portion de cône, et donc seulement sur une partie de la hauteur du capuchon, force l'utilisateur à cibler son action de rotation à la base de l'embout, proche du dispositif de retenue, ce qui permet de faciliter la fracture des picots formant ce dispositif de retenue.

On comprendra que si les différents modes de réalisation ont été décrits avec un embout de forme conique, partiel ou non, on pourra sans sortir du contexte de l'invention proposer une forme externe différente étant entendu que la forme interne doit dans tous les cas permettre le recouvrement de l'embout de distribution et du capuchon associé.

Par ailleurs, on peut observer que les moyens de guidage axial des deux parties du manchon de recouvrement 240, 242 diffèrent du mode de réalisation précédent en ce que la partie supérieure 240 comporte une lumière 64 non débouchante sur son bord inférieur comme pouvait l'être la rainure 46 précédemment. L'ergot 248 porté par la partie inférieure 242 du manchon de recouvrement présente alors une élasticité qui lui permet de se déformer pour passer au-delà du bord inférieur de la partie supérieure lorsqu'on les rapproche l'une de l'autre et venir se loger dans la lumière. On observe qu'on forme ainsi un moyen de retenue axial des deux parties de recouvrement l'une par rapport à l'autre.

Le flacon de conditionnement de liquide tel qu'il vient d'être décrit dans chacun de ces modes de réalisation présente l'avantage de pouvoir être fabriqué par des étapes successives bien distinctes. Dans un premier temps, on assemble un réservoir et une tête de distribution porteuse d'un système de pompe de prélèvement, d'une portion mobile axialement d'actionnement du système, et d'un embout de distribution solidaire en déplacement de la portion mobile et par lequel le liquide est délivré. Et on réalise dans des conditions stériles la fermeture de l'embout de distribution par un capuchon étanche et le remplissage de liquide du réservoir avant la fermeture étanche dudit réservoir.

Avantageusement, les étapes suivantes de montage de la coiffe, fabriquée par ailleurs, peuvent alors être effectuées dans des conditions non nécessairement stériles. On enfile la coiffe autour du flacon. La partie amovible, retenue au manchon de recouvrement par le dispositif de retenue fracturable, est disposée dans le même mouvement autour du flacon, prête à être enlevée par fracture du dispositif de retenue, avant la première utilisation. Tel que cela a pu être compris précédemment, on dispose alors d'un témoin de première utilisation du flacon avec cette partie amovible. L'utilisateur sait que le flacon qu'il a devant lui a déjà été utilisé si la partie amovible a été retirée ou si la partie amovible n'est plus retenue par le dispositif fracturable.

## Revendications

1. Flacon de conditionnement de liquide comportant un réservoir (2;102) sur lequel est montée une tête de distribution (4;104), laquelle comporte une portion (16) mobile axialement d'un système de pompe de prélèvement du liquide et un embout de distribution (12) solidaire en déplacement de ladite portion mobile de pompe et par lequel le liquide est délivré, ladite portion mobile présentant une collerette (18) sur une face supérieure de laquelle est fixé ledit embout de distribution, **caractérisé en ce qu'**il comporte une coiffe (6;106) formée d'un manchon (20;40,42;140,142) de recouvrement de ladite portion mobile pour l'actionnement de la pompe et d'une partie amovible (22;122;222) de recouvrement du flacon, ledit manchon comportant une paroi latérale cylindrique (26;126) dont l'extrémité supérieure est prolongée par une paroi transversale (28;128) qui est adaptée à reposer sur ladite face supérieure de ladite collerette et qui présente un alésage central (30) permettant le passage de l'embout, ladite partie amovible de la coiffe étant retenue à ladite paroi transversale dudit manchon par un dispositif de retenue (24;124) fracturable lors de la première utilisation du flacon, de sorte que ladite partie amovible de la coiffe recouvre l'alésage central, à l'opposé de ladite paroi latérale du manchon (26; 126), lorsqu'elle est retenue à ladite paroi transversale (28, 128) du manchon, et que l'alésage central et l'embout de distribution sont dégagés lorsque la partie amovible de la coiffe (22, 122, 222) est retirée après fracture du dispositif de retenue.

2. Flacon selon la revendication 1, **caractérisé en ce que** le dispositif de retenue de ladite partie amovible de la coiffe à ladite paroi transversale du manchon (28;128) est constitué par des picots de maintien (25) fracturables par rotation de la partie amovible de la coiffe (22, 122, 222) par rapport au manchon lors de la première utilisation du flacon.

3. Flacon selon la revendication 2, **caractérisé en ce que** les picots fracturables (25) sont disposés transversalement entre l'extrémité de ladite partie amovible de la coiffe et le bord du manchon délimitant l'alésage central, dans le plan de la collerette d'actionnement de la pompe.

4. Flacon selon l'une des revendications précédentes, **caractérisé en ce que** la partie amovible de la coiffe présente une forme d'embout conique (50;150) complémentaire de la forme de l'embout de distribution (12) et d'un capuchon (36) venant le recouvrir, ladite partie amovible comportant des bossages (56) sur la face intérieure de la paroi délimitant sa forme conique.

5. Flacon selon la revendication précédente, **caractérisé en ce que** l'embout conique formant la partie amovible de la coiffe présente une forme de portion de cône (250) duquel dépasse ledit capuchon (36) en saillie de son extrémité (60) à l'opposé de la paroi transversale du manchon.

6. Flacon selon la revendication précédente, **caractérisé en ce que** ladite portion de cône (250) présente sur sa face externe des nervures axiales (62) de rigidification.

7. Flacon selon l'une des revendications précédentes, **caractérisé en ce que** ladite coiffe (6;106) comporte des moyens d'encliquetage (34) disposés sur la face intérieure de ladite paroi latérale (26;126) dudit manchon, pour l'emprisonnement de ladite collerette (18) entre ces moyens d'encliquetage et la paroi transversale du manchon (28;128).

8. Flacon selon l'une des revendications précédentes, **caractérisé en ce que** la paroi transversale du manchon (28;128) est prolongée radialement, à l'extérieur du manchon, par une ailette de préhension (32;132).

9. Flacon selon l'une des revendications précédentes, **caractérisé en ce que** la paroi latérale du manchon (26;126) s'étend axialement pour recouvrir ladite collerette (18) et au moins une partie du réservoir (2;102).

10. Flacon selon la revendication 9, **caractérisé en ce que** la paroi transversale (28;128) du manchon est prolongée radialement à l'extérieur du manchon par une ailette de préhension (32;132) et **en ce que** la paroi latérale (26;126) du manchon s'étend axialement le long du réservoir (2;102) sous ladite ailette, le flacon comportant des moyens de blocage en rotation de la coiffe relativement au flacon qui sont adaptés à ce que, dans la position angulaire bloquée de la coiffe, la paroi latérale du manchon s'étende le long du réservoir sous ladite ailette de préhension et sur un côté du flacon opposé à un côté réservé à l'étiquetage du flacon.

11. Flacon selon l'une des revendications précédentes, dans lequel ledit manchon de recouvrement du flacon comporte deux parties coulissant l'une par rapport à l'autre, une partie supérieure (40;240) de recouvrement de ladite portion mobile pour l'actionnement de la pompe et une partie inférieure (42;242) recouvrant le fond du réservoir, les deux parties présentant des ailettes radiales (132) de préhension du flacon, et dans lequel les deux parties coulissantes sont bloquées en rotation relative l'une par rapport à l'autre, ladite partie amovible de la coiffe étant rapportée sur la partie supérieure du manchon par un dispositif de retenue (24) fracturable par rotation de la partie amovible de la coiffe par rapport au manchon lors de la première utilisation du flacon.

12. Flacon suivant la revendication 11, **caractérisé en ce qu'**il comporte un moyen (64 - 248) de retenue axiale des deux parties de recouvrement l'une par rapport à l'autre.

13. Procédé de fabrication d'un flacon de conditionnement de liquide suivant l'une quelconque des revendications 1 à 12, dans lequel :
- on réalise dans des conditions stériles le remplissage du réservoir (2;102) par du liquide puis l'assemblage étanche de ce réservoir (2;102) avec la tête de distribution du liquide (4;104), ladite tête étant porteuse du système de pompe de prélèvement de liquide, de ladite portion mobile axialement (16) d'actionnement du système de pompe, dudit embout de distribution de liquide (12) solidaire en déplacement de ladite portion mobile, ainsi que d'un capuchon (36;136;236) recouvrant ledit embout ;
et
- dans un deuxième temps, dans des conditions non nécessairement stériles, on dispose ladite coiffe (6;106), formée dudit manchon en recouvrement de ladite portion mobile pour l'actionnement du système de pompe et de ladite partie amovible de la coiffe, retenue audit manchon par ledit dispositif de retenue fracturable (24), en recouvrement du flacon et dudit capuchon (36;136;236) recouvrant l'embout.

## Patentansprüche

1. Fläschchen für die Verpackung von Flüssigkeit, das einen Vorratsbehälter (2; 102) umfasst, auf dem ein Verteilerkopf (4; 104) montiert ist, der einen axial beweglichen Abschnitt (16) eines Pumpsystems für die Entnahme von Flüssigkeit und ein Verteilerendstück (12), das verlagerungsfest mit dem beweglichen Pumpabschnitt verbunden ist und durch den die Flüssigkeit ausgegeben wird, enthält, wobei der bewegliche Abschnitt einen Kranz (18) aufweist, auf dessen Oberfläche das Verteilerendstück befestigt ist, **dadurch gekennzeichnet, dass** es eine Kappe (6; 106) umfasst, die aus einer Muffe (20; 40, 42; 140, 142) für den beweglichen Abschnitt für die Betätigung der Pumpe und aus einem abnehmbaren Teil (22; 122; 222) für die Abdeckung des Fläschchens gebildet ist, wobei die Muffe eine zylindrische Seitenwand (26; 126) aufweist, deren oberes Ende durch eine transversale Wand (28; 128) verlängert ist, die dafür ausgelegt ist, auf der oberen Fläche des Kranzes aufzuruhen, und die eine Mittelbohrung (30) aufweist, die den Durchgang des Endstücks ermöglicht, wobei der abnehmbare Teil der Kappe an der transversalen Wand der Muffe durch eine Haltevorrichtung (24; 124) gehalten wird, die bei der ersten Verwendung des Fläschchens zerbrochen werden kann, derart, dass der abnehmbare Teil der Kappe die Mittelbohrung gegenüber der Seitenwand der Muffe (26; 126) abdeckt, wenn er an der transversalen Seitenwand (28, 128) der Muffe gehalten wird, und dass die Mittelbohrung und das Verteilerendstück voneinander gelöst sind, wenn der abnehmbare Teil der Kappe (22, 122, 222) nach dem Zerbrechen der Haltevorrichtung abgezogen worden ist.

2. Fläschchen nach Anspruch 1, **dadurch gekennzeichnet, dass** die Vorrichtung zum Halten des abnehmbaren Teils der Kappe an der transversalen Seitenwand der Muffe (28; 128) durch Haltestacheln (25) gebildet ist, die durch Drehen des abnehmbaren Teils der Kappe (22, 122, 222) in Bezug auf die Muffe bei der ersten Verwendung des Fläschchens zerbrochen werden können.

3. Fläschchen nach Anspruch 2, **dadurch gekennzeichnet, dass** die zerbrechbaren Stacheln (25) in Querrichtung zwischen dem Ende des abnehmbaren Teils der Kappe und dem Rand der Muffe, der die Mittelbohrung begrenzt, in der Ebene des Kranzes für die Betätigung der Pumpe angeordnet sind.

4. Fläschchen nach einem der vorhergehenden Ansprüche, **dadurch gekennzeichnet, dass** der abnehmbare Teil der Kappe die Form eines konischen Endstücks (50; 150), die zu der Form des Verteilerendstücks (12) komplementär ist, und einer Abdeckkappe (36), die es abdeckt, aufweist, wobei der abnehmbare Teil auf der Innenfläche der Wand, die seine konische Form begrenzt, Höcker aufweist.

5. Fläschchen nach dem vorhergehenden Anspruch, **dadurch gekennzeichnet, dass** das konische Endstück, das den abnehmbaren Teil der Kappe bildet, die Form eines Konusabschnitts (250) besitzt, den die Abdeckkappe (36) an seinem Ende (60) gegenüber der transversalen Wand der Muffe überragt.

6. Fläschchen nach dem vorhergehenden Anspruch, **dadurch gekennzeichnet, dass** der Konusabschnitt (250) auf seiner äußeren Fläche axiale Versteifungsrippen (62) aufweist.

7. Fläschchen nach einem der vorhergehenden Ansprüche, **dadurch gekennzeichnet, dass** die Kappe (6; 106) Einrastmittel (34) umfasst, die auf der inneren Fläche der Seitenwand (26; 126) der Muffe angeordnet sind, um den Kranz (18) zwischen diesen Einrastmitteln und der transversalen Wand der Muffe (28; 128) einzuschließen.

8. Fläschchen nach einem der vorhergehenden Ansprüche, **dadurch gekennzeichnet, dass** die transversale Wand der Muffe (28; 128) durch einen Greifflügel (32; 132) in die äußere Umgebung der Muffe radial verlängert ist.

9. Fläschchen nach einem der vorhergehenden Ansprüche, **dadurch gekennzeichnet, dass** sich die Seitenwand der Muffe (26; 126) axial erstreckt, um den Kranz (18) und wenigstens einen Teil des Vorratsbehälters (2; 102) abzudecken.

10. Fläschchen nach Anspruch 9, **dadurch gekennzeichnet, dass** die transversale Wand (28; 128) der Muffe durch einen Greifflügel (32; 132) in die äußere Umgebung der Muffe radial verlängert ist und dass sich die Seitenwand (26; 126) der Muffe axial längs des Vorratsbehälters (2; 102) unter dem Flügel erstreckt, wobei das Fläschchen Drehblockiermittel für die Kappe relativ zu dem Fläschchen umfasst, die so beschaffen sind, dass sich die Seitenwand der Muffe in der blockierten Winkelposition der Kappe längs des Vorratsbehälters unter dem Greifflügel und auf einer Seite des Fläschchens gegenüber einer der Etikettierung des Fläschchens vorbehaltenen Seite erstreckt.

11. Fläschchen nach einem der vorhergehenden Ansprüche, wobei die Muffe zum Abdecken des Fläschchens zwei relativ zueinander gleitende Teile umfasst, nämlich einen oberen Teil (40; 240) für die Abdeckung des beweglichen Abschnitts für die Betätigung der Pumpe und einen unteren Teil (42; 242, der den Boden des Vorratsbehälters abdeckt, wobei die beiden Teile radiale Flügel (132) zum Ergreifen des Fläschchens aufweisen, und wobei die beiden gleitenden Teile in Drehrichtung relativ zueinander blockiert sind, wobei der abnehmbare Teil der Kappe an den oberen Teil der Muffe durch eine Haltevorrichtung (24) angefügt ist, die durch Drehen des abnehmbaren Teils der Kappe in Bezug auf die Muffe bei der ersten Verwendung des Fläschchens zerbrochen werden kann.

12. Fläschchen nach Anspruch 11, **dadurch gekennzeichnet, dass** es ein Mittel (64-248) zum axialen Halten der beiden Abdeckteile relativ zueinander umfasst.

13. Verfahren zum Herstellen eines Fläschchens für die Verpackung von Flüssigkeit nach einem der Ansprüche 1 bis 12, das Folgendes umfasst:
- Ausführen unter sterilen Bedingungen des Befüllens des Vorratsbehälters (2; 102) mit der Flüssigkeit und anschließend dichtes Zusammenfügen dieses Vorratsbehälters (2; 102) mit dem Verteilerkopf (4; 104) für die Flüssigkeit, wobei der Kopf das Pumpsystem für die Entnahme von Flüssigkeit, den axial beweglichen Abschnitt (16) für die Betätigung des Pumpsystems, das Verteilerendstück (12) für die Flüssigkeit, das mit dem beweglichen Abschnitt verlagerungsfest verbunden ist, sowie eine Abdeckkappe (36; 136; 236), die das Endstück abdeckt, trägt; und
- zu einer zweiten Zeit unter nicht unbedingt sterilen Bedingungen Anordnen der Kappe (6; 106), die aus der Muffe zum Abdecken des beweglichen Abschnitts für die Betätigung des Pumpsystems und aus dem abnehmbaren Teil der Kappe, der an der Muffe durch die zerbrechbare Haltevorrichtung (24) gehalten wird, gebildet ist, wobei das Fläschchen und die Abdeckkappe (36; 136; 236), die das Endstück abdeckt, abgedeckt werden.

## Claims

1. Bottle for packaging liquid, comprising a reservoir (2; 102) on which there is mounted a dispensing head (4; 104) that comprises an axially movable portion (16) of a liquid-takeoff pump system and a dispensing nozzle (12) constrained to move with said movable pump portion and through which the liquid is delivered, said movable portion having a collar (18), to a top face of which said dispensing nozzle is fixed, **characterised in that** it comprises a cover (6, 106) formed by a sleeve (20; 40, 42; 140, 142) covering said movable portion for actuating the pump and a removable part (22; 122; 222) for covering the bottle, said sleeve comprising a cylindrical lateral wall (26; 126), the top end of which is extended by a transverse wall (28; 128) that is suitable for resting on said top face of said collar and which has a central bore (30) enabling the nozzle to pass, said removable part of the cover being held on said transverse wall of said sleeve by a holding device (24; 124) that can be broken when the bottle is first used, so that said removable part of the cover covers the central bore, opposite said lateral wall of the sleeve (26; 126), when it is held on said transverse wall (28; 128) of the sleeve, and so that the central bore and the dispensing nozzle are released when the removable part of the cover (22, 122, 222) is removed after breakage of the holding device.

2. Bottle according to claim 1, **characterised in that** the device holding said removable part of the cover on said transverse wall of the sleeve (28, 128) is formed by holding spikes (25) that can be broken by rotation of the removable part of the cover (22, 122, 222) with respect to the sleeve when the bottle is first used.

3. Bottle according to claim 2, **characterised in that** the breakable spikes (25) are disposed transversely between the end of said removable part of the cover and the edge of the sleeve delimiting the central bore, in the plane of the collar actuating the pump.

4. Bottle according to any of the preceding claims, **characterised in that** the removable part of the cover has a conical end-piece form (50, 150) complementary to the form of the dispensing nozzle (12) and a cap (36) covering it, said removable part comprising protrusions (56) on the internal face of the wall delimiting its conical form.

5. Bottle according to the preceding claim, **characterised in that** the conical end piece forming the removable part of the cover has a cone-portion shape (250) beyond which said cap (36) protrudes, projecting from its end (60) opposite to the transverse wall of the sleeve.

6. Bottle according to the preceding claim, **characterised in that** said cone portion (250) has axial stiffening ribs (62) on its external face.

7. Bottle according to any of the preceding claims, **characterised in that** said cover (6; 106) comprises snap-on means (34) disposed on the internal face of said lateral wall (26; 126) of said sleeve, for trapping said collar (18) between these snap-on means and the transverse wall of the sleeve (28; 128).

8. Bottle according to any of the preceding claims, **characterised in that** the transverse wall of the sleeve (28; 128) is extended radially, outside the sleeve, by a gripping fin (32; 132).

9. Bottle according to any of the preceding claims, **characterised in that** the lateral wall of the sleeve (26; 126) extends axially so as to cover said collar (18) and at least part of the reservoir (2; 102).

10. Bottle according to claim 8, **characterised in that** the transverse wall (28; 128) of the sleeve is extended radially outside the sleeve by a gripping fin (32; 132) and **in that** the lateral wall (26; 126) of the sleeve extends axially along the reservoir (2; 102) under said fin, the bottle comprising means for locking the cover with respect to rotation relative to the bottle that are adapted so that, in the locked angular position of the cover, the lateral wall of the sleeve extends along the reservoir under said gripping fin and on a side of the bottle opposite to a side reserved for snapping on the bottle.

11. Bottle according to any of the preceding claims, in which said sleeve covering the bottle comprises two parts sliding with respect to one another, a top part (40; 240) covering said movable portion for actuating the pump and a bottom part (42; 242) covering the bottom of the reservoir, the two parts having radial fins (132) for gripping the bottle, and wherein the two sliding parts are locked with respect to rotation relative to one another, said removable part of the cover being attached to the top part of the sleeve by a holding device (24) that can be broken by rotation of the removable part of the cover with respect to the sleeve when the bottle is first used.

12. Bottle according to claim 11, **characterised in that** it comprises a means (64-248) for axial holding of the two covering parts with respect to one another.

13. Method for manufacturing a liquid-packaging bottle according to any of claims 1 to 12, wherein:
- under sterile conditions, the reservoir (2; 102) is filled with liquid and then this reservoir (2; 102) is assembled sealingly with the liquid-dispensing head (4; 104), said head carrying the liquid-takeoff pump system, said axially movable portion (16) for actuating the pump system, said liquid-dispensing nozzle (12) constrained to move with said movable portion, and a cap (36; 136; 236) covering said nozzle; and
- secondly, under conditions that are not necessarily sterile, said cover (6; 106) is disposed, formed by said sleeve covering said movable portion for actuating the pump system and said removable part of the cover, held on said sleeve by said breakable holding device (24), covering the bottle and said cap (36; 136; 236) covering the nozzle.
